# EUROPÄISCHE PATENTSCHRIFT

(11) **EP 0 796 271 B1**
(45) Veröffentlichungstag und Bekanntmachung des Hinweises auf die Patenterteilung: **02.02.2000**
(21) Anmeldenummer: 95941015.0
(22) Anmeldetag: 25.11.1995
(51) Int. Cl.: C07K 5/06, A61K 38/55

(54) **DIPEPTIDISCHE P-AMIDINOBENZYLAMIDE MIT N-TERMINALEN SULFONYL- BZW. AMINOSULFONYLRESTEN**
DIPEPTIDE P-AMIDINOBENZYLAMIDES WITH N-TERMINAL SULFONYL OR AMINOSULFONYL RADICALS
P-AMIDINOBENZYLAMIDES DIPEPTIDIQUES A RESTES N-TERMINAUX SULFONYLE OU AMINOSULFONYLE

(30) Priorität: 06.12.1994 DE 4443390
(43) Veröffentlichungstag der Anmeldung: 24.09.1997
(73) Patentinhaber: BASF AKTIENGESELLSCHAFT, 67056 Ludwigshafen (DE)
(72) Erfinder: BÖHM, Hans Joachim, 67117 Limburgerhof (DE); KOSER, Stefan, 67063 Ludwigshafen (DE); MACK, Helmut, 67067 Ludwigshafen (DE); PFEIFFER, Thomas, 67459 Böhl-Iggelheim (DE); SEITZ, Werner, 68723 Plankstadt (DE); HÖFFKEN, Hans Wolfgang, 67069 Ludwigshafen (DE); HORNBERGER, Wilfried, 67434 Neustadt (DE); ZIERKE, Thomas, 67459 Böhl-Iggelheim (DE)
(86) Internationale Anmeldenummer: EP9504646
(87) Internationale Veröffentlichungsnummer: WO9617860

(56) Entgegenhaltungen:
- EP-A- 0 601 459
- EP-A- 0 669 317
- EP-A- 0 672 658
- WO-A-93/11152
- WO-A-94/29336

## Beschreibung

Die vorliegende Erfindung betrifft dipeptidische p-Amidinobenzylamide mit N-terminalen Sulfonyl- bzw. Aminosulfonylresten, ihre Herstellung und ihre Verwendung als Thrombininhibitoren.

WO 93/11152 beschreibt lineare amidinohaltige Thrombininhibitoren, die auch eine Sulfonamidgruppe enthalten können.

EP 601 459 beschreibt lineare und heterocyclische Thrombininhibitoren, die eine Sulfonamidgruppe aufweisen.

Die vorliegende Erfindung betrifft Verbindungen der Formel I 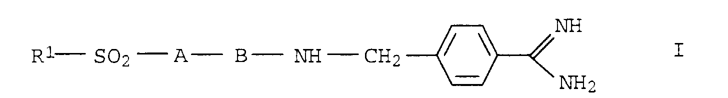 sowie deren Stereoisomeren und deren Salze mit physiologisch verträglichen Säuren, worin die Substituenten folgende Bedeutungen besitzen:
- R¹: C₁-C₂₀-Alkyl, C₁-C₃-Fluoralkyl, C₃-C₈-Cycloalkyl, Aryl-C₁-C₁₀-alkyl, Aryl, Heteroaryl, OH oder R³R²N, wobei R² und R³ gleich oder verschieden sind und Wasserstoff, C₁-C₁₀-Alkyl, Aryl, Aryl-C₁-C₁₀-Alkyl oder zusammen eine C₂-C₇-Alkylenkette, an die gegebenenfalls ein Aryl- oder Heteroarylrest ankondensiert ist oder die ein Heteroatom (O, S, NH bzw. substituiertes N) enthalten kann, bedeuten,
- A: ein α-Aminosäurerest der Formel II 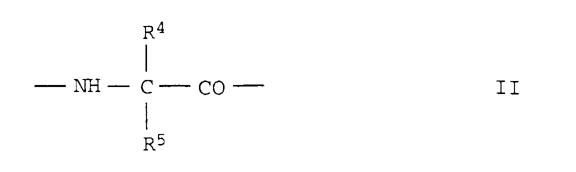 worin
R⁴ Wasserstoff, C₁-C₈-Alkyl, C₃-C₇-Cycloalkyl, Aryl oder Aryl-C₁-C₃-alkyl,
R⁵ Wasserstoff, C₁-C₈-Alkyl, C₃-C₇-Cycloalkyl, C₃-C₇-Cycloalkyl-C₁₋₃-alkyl, Aryl, Aryl-C₁-C₃-alkyl, Di(C₃-C₇-Cycloalkyl)-methyl oder Diphenylmethyl oder - falls R⁴ = H - einen C₁-C₈-Alkylrest, in dem ein Wasserstoffatom durch OR⁶ oder CO-OR⁶ (R⁶ = Wasserstoff, C₁-C₈-Alkyl oder Aryl-C₁-C₃-alkyl) ersetzt ist oder
R⁴ und R⁵ zusammen eine C₂-C₆-Alkylenkette, die einen ankondensierten Arylrest enthalten kann, bedeuten,
- B: ein cyclischer α-Aminosäurerest der Formel III 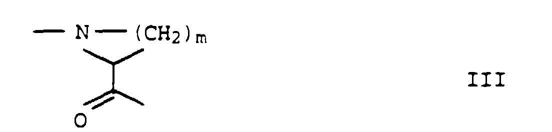 worin m die Zahl 2, 3 oder 4 bedeutet und ein Wasserstoff am Ring durch eine Hydroxy- oder C₁-C₃-Alkylgruppe und - falls m = 3 oder 4 ist - eine CH₂-Gruppe im Ring durch Sauerstoff, Schwefel, NH- oder N-C₁-C₄-Alkyl und/oder zwei benachbarte Wasserstoffatome durch eine Doppelbindung ausgetauscht sein können.

Bevorzugt sind die folgenden vier Verbindungsgruppen:
1. Verbindungen der Formel I, worin die Substituenten folgende Bedeutungen besitzen:
   - R¹: OH oder R³R²N, wobei R² und R³ gleich oder verschieden sind und Wasserstoff, C₁-C₁₀-Alkyl, Aryl, Aryl-C₁-C₁₀-alkyl oder zusammen eine C₂-C₇-Alkylenkette, an die gegebenenfalls ein Aryl- oder Heteroarylrest ankondensiert ist oder die ein Heteroatom (O, S, NH bzw. substituiertes N) enthalten kann, bedeuten,
   - A: ein α-Aminosäurerest der Formel II 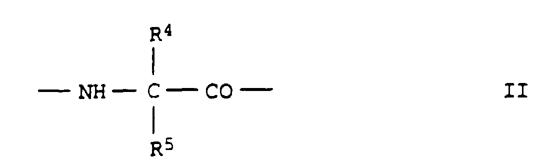 worin
   R⁴ Wasserstoff, C₁-C₈-Alkyl, C₃-C₇-Cycloalkyl, Aryl oder Aryl-C₁-C₃-alkyl,
   R⁵ Wasserstoff, C₁-C₈-Alkyl, C₃-C₇-Cycloalkyl, C₃-C₇-Cycloalkyl-C₁₋₃-alkyl, Aryl, Aryl-C₁-C₃-alkyl, Di(C₃-C₇-Cycloalkyl)-methyl oder Diphenylmethyl oder - falls R⁴ = H - einen C₁-C₈-Alkylrest, in dem ein Wasserstoffatom durch OR⁶ oder CO-OR⁶ (R⁶ = Wasserstoff, C₁-C₈-Alkyl oder Aryl-C₁-C₃-alkyl) ersetzt ist oder
   R⁴ und R⁵ zusammen eine C₂-C₆-Alkylenkette, die einen ankondensierten Arylrest enthalten kann, bedeuten,
   - B: ein cyclischer α-Aminosäurerest der Formel III 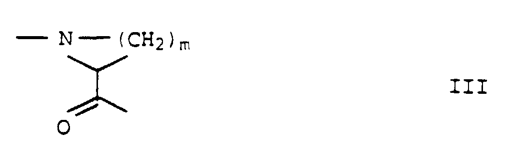 worin m die Zahl 2, 3 oder 4 bedeutet und ein Wasserstoff am Ring durch eine Hydroxy- oder C₁-C₃-Alkylgruppe und - falls m = 3 oder 4 ist - eine CH₂-Gruppe im Ring durch Sauerstoff, Schwefel, NH- oder N-C₁-C₄-Alkyl und/oder zwei benachbarte Wasserstoffatome durch eine Doppelbindung ausgetauscht sein können.
2. Verbindungen der Formel I, worin die Substituenten folgende Bedeutungen besitzen:
   - R¹: C₁-C₂₀-Alkyl, C₁-C₃-Fluoralkyl, C₃-C₈-Cycloalkyl, Aryl-C₁-C₁₀-alkyl, Aryl oder Heteroaryl bedeuten,
   - A: ein α-Aminosäurerest der Formel II 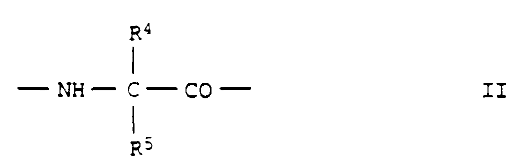 worin
   R⁴ C₁-C₈-Alkyl, C₃-C₇-Cycloalkyl, Aryl oder Aryl-C₁-C₃-alkyl,
   R⁵ C₁-C₈-Alkyl, C₃-C₇-Cycloalkyl, C₃-C₇-Cycloalkyl-C₁₋₃-alkyl, Aryl, Aryl-C₁-C₃-alkyl, Di(C₃-C₇-Cycloalkyl)-methyl oder Diphenylmethyl oder
   R⁴ und R⁵ zusammen eine C₂-C₆-Alkylenkette, die einen ankondensierten Arylrest enthalten kann, bedeuten,
   - B: einer der Reste 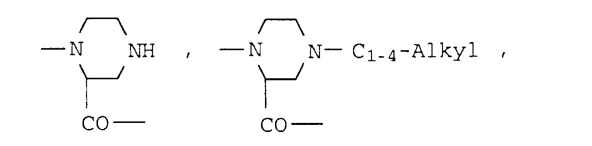 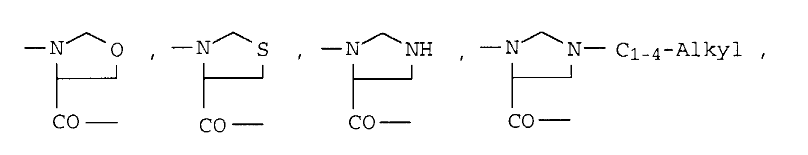 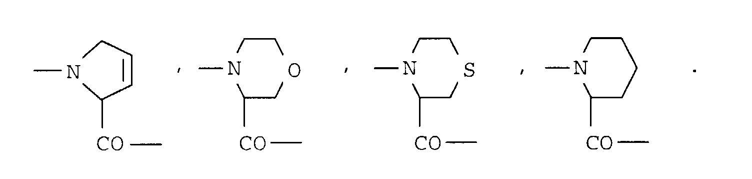
3. Verbindungen der Formel I, worin die Substituenten folgende Bedeutungen besitzen:
   - R¹: C₁-C₂₀-Alkyl, C₁-C₃-Fluoralkyl, C₃-C₈-Cycloalkyl, Aryl-C₁-C₁₀-alkyl, Aryl, Heteroaryl oder OH bedeuten,
   - A: ein α-Aminosäurerest der Formel 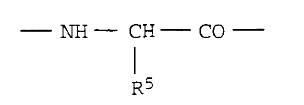 worin
   R⁵ einen C₁-C₈-Alkylrest, in dem ein Wasserstoffatom durch OR⁶ oder CO-OR⁶ (R⁶ = Wasserstoff, C₁-C₈-Alkyl oder Aryl-C₁-C₃-alkyl) ersetzt ist, bedeutet,
   - B: einer der Reste 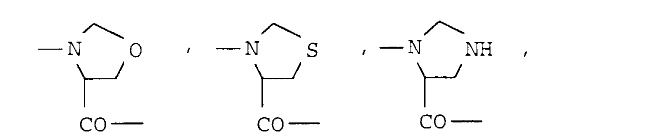 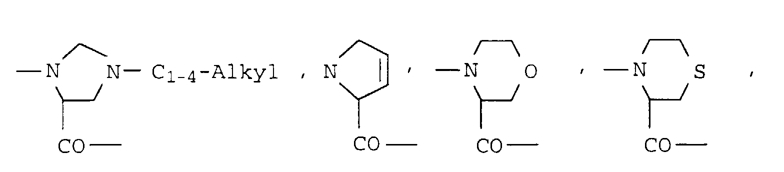 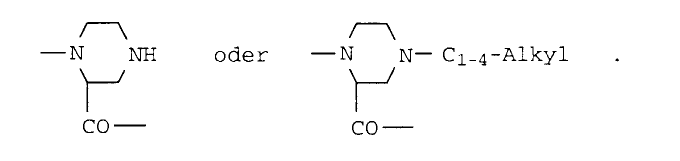
4. Verbindungen der Formel I, worin die Substituenten folgende Bedeutungen besitzen:
   - R¹: C₁-C₂₀-Alkyl, C₁-C₃-Fluoralkyl, C₃-C₈-Cycloalkyl, Aryl-C₁-C₁₀-alkyl, Aryl, Heteroaryl oder OH bedeuten,
   - A: ein α-Aminosäurerest der Formel 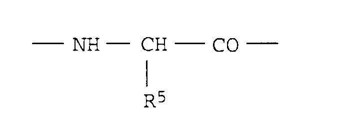 worin
   R⁵ einen C₁-C₈-Alkylrest, in dem ein Wasserstoffatom durch OR⁶ oder CO-OR⁶ (R⁶ = Wasserstoff oder C₁-C₈-Alkyl) ersetzt ist, bedeutet,
   - B: ein cyclischer α-Aminosäurerest der Formel III 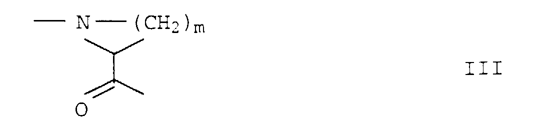 worin m die Zahl 2, 3 oder 4 bedeutet und ein Wasserstoff am Ring durch eine Hydroxy- oder C₁-C₃-Alkylgruppe ausgetauscht ist.

Der Begriff "Aryl" bezeichnet mono- oder bicyclische aromatische Gruppen, die 6 bis 10 Kohlenstoffatome im Ringsystem enthalten, z.B. Phenyl oder Naphthyl, und mit bis zu drei gleichen oder verschiedenen Substituenten versehen sein konnen.

Die Bezeichnung "Heteroaryl" bezieht sich auf 5- oder 6-gliedrige aromatische Ringe, die 1 oder 2 Heteroatome wie N, O oder S enthalten können und an die ein Arylring ankondensiert sein kann.

In den Gruppen 1-4 der Verbindungen I sind Verbindungen mit folgenden Substituenten R¹, A und B besonders zu nennen:

### Verbindungsgruppe 1

- R¹: NH₂, C₁₋₄-Mono- bzw. Dialkylamino oder Piperidinyl
- A: Reste der Aminosauren Valin, Leucin, Isoleucin, Phenylglycin, Cyclohexylglycin, Phenylalanin, Cyclohexylalanin, Diphenylalanin, Dicyclohexylalanin, wobei in den Resten vorhandene Phenylringe durch bis zu drei gleiche oder verschiedene C₁₋₄-Alkyl-, C₁₋₄-Alkoxy, OH-, F-, Cl- oder COOR⁶-Gruppen substituiert sein können
- B: Reste der Aminosäuren Azetidin-2-carbonsäure, Prolin, Pipecolinsäure, Dehydroprolin, 4-Hydroxyprolin, Oxaprolin, Thiaprolin, 4-C₁₋₄-Alkyl-pipecolinsäure, Morpholin-2-carbonsäure, Piperazin-2-carbonsaure, 4-C₁₋₄-Alkyl-piperazin-2-carbonsäure

### Verbindungsgruppe 2

- R¹: C₁₋₄-Alkyl, CF₃-CH₂-, Phenyl, Naphthyl, Phenyl-C₁₋₄-alkylen (besonders Benzyl und Phenethyl), Naphthyl-C₁₋₄-alkylen, Pyridyl, Isochinolyl
- A: Reste der Aminosäuren α-Methyl-phenylalanin, α-Methylcyclohexylalanin, α-Methyl-phenylglycin, α-Methyl-cyclohexylglycin
- B: Reste der Aminosäuren Dehydroprolin, Oxaprolin, Thiaprolin, Pipecolinsäure, 4-C₁₋₄-Alkyl-pipecolinsäure, Morpholin-2-carbonsäure, Piperazin-2-carbonsäure, 4-C₁₋₄-Alkyl-piperazin-2-carbonsäure

### Verbindungsgruppe 3

- R¹: C₁₋₄-Alkyl, CF₃-CH₂-, Phenyl, Naphthyl, Phenyl-C₁₋₄-alkylen (besonders Benzyl und Phenethyl), Naphthyl-C₁₋₄-alkylen, Pyridyl, Isochinolyl
- A: Reste der Aminosäuren Asparaginsäure, Glutaminsäure, Serin, Homoserin, Threonin, wobei die nicht verknüpfte Carbonsäure bzw. die Hydroxygruppe durch einen C₁₋₈-Alkylrest verestert bzw. verethert (besonders t-Butyl-Serin und t-Butyl-threonin) sein kann
- B: Reste der Aminosäuren Dehydroprolin, Oxaprolin,Thiaprolin, Morpholin-2-carbonsäure

### Verbindungsgruppe 4

- R¹: C₁₋₄-Alkyl, CF₃-CH₂-, Phenyl, Naphthyl, Phenyl-C₁₋₄-alkylen (besonders Benzyl und Phenethyl), Naphthyl-C₁₋₄-alkylen, Pyridyl, Isochinolyl
- A: Reste der Aminosäuren Serin, Homoserin, Threonin, in denen die OH-Gruppe durch C₁₋₈-Alkyl verethert sein kann oder Asparaginsäure, Glutaminsäure, in denen die nicht verknüpfte Carbonsäure durch C₁₋₈-Alkyl verestert sein kann
- B: Reste der Aminosäuren 4-Hydroxyprolin, 4-C₁₋₄-Alkyl-pipecolinsäure

Die Reste R¹, A und B sind wie in Struktur I dargestellt miteinander verknupft, wobei die Aminosaurereste in A bevorzugt (D)-konfiguriert und die Aminosäurereste in B bevorzugt (L)-konfiguriert sind.

Folgende Substanzen seien vorzugsweise genannt:
CF₃-CH₂-SO₂-(D)Phe-Pro-NH-pAmb
C₄H₉-SO₂-(D)Phe-Pro-NH-pAmb
C₈H₁₇-SO₂-(D)Phe-Pro-NH-pAmb
C₁₆H₃₃-SO₂-(D)Phe-Pro-NH-pAmb
i-Propyl-SO₂-(D)Phe-Pro-NH-pAmb
Phenyl-SO₂-(D)Phe-Pro-NH-pAmb
2-Naphthyl-SO₂-(D)Phe-Pro-NH-pAmb
3-Pyridyl-SO₂-(D)Phe-Pro-NH-pAmb
2-Thienyl-SO₂-(D)Phe-Pro-NH-pAmb
N-Piperidinyl-SO₂-(D)Phe-Pro-NH-pAmb
H₂N-SO₂-(D)Phe-Pro-NH-pAmb
Me₂N-SO₂-(D)Phe-Pro-NH-pAmb
EtHN-SO₂-(D)Phe-Pro-NH-pAmb
Me-SO₂-(D)Phe (4-OMe)-Pro-NH-pAmb
Me-SO₂-(D)Phe(3-OMe)-Pro-NH-pAmb
Me-SO₂-(D)Phe(2-Cl)-Pro-NH-pAmb
Me-SO₂-(D)Dpa-Pro-NH-pAmb
Me-SO₂-(L)Dpa-Pro-NH-pAmb
Me-SO₂-(D)Dpa(4,4'-OMe)-Pro-NH-pAmb
Me-SO₂-(L)Dpa(4,4'-OMe)-Pro-NH-pAmb
Me-SO₂-(D)Dpa(4,4'-Cl)-Pro-NH-pAmb
Me-SO₂-(L)Dpa(4,4'-Cl)-Pro-NH-pAmb
Me-SO₂-(D,L)Phg(3,4-Cl)-Pro-NH-pAmb
Me-SO₂-(D)Asp(OH)-Pro-NH-pAmb
Me-SO₂-(L)Asp(OH)-Pro-NH-pAmb
Me-SO₂-(D)Asp(OMe)-Pro-NH-pAmb
Me-SO₂-(L)Asp(OMe)-Pro-NH-pAmb
Me-SO₂-(D)Asp(OtBu)-Pro-NH-pAmb
Me-SO₂-(L)Asp(OtBu)-Pro-NH-pAmb
Me-SO₂-(D)Phe-Aze-NH-pAmb
Me-SO₂-(D)Phe-Pip-NH-pAmb
1-Naphthyl-SO₂-Gly-Pro-NH-pAmb

Die in dieser Übersicht verwendeten Abkürzungen haben folgende Bedeutungen:
Phe = Phenylalanin, pAmb = p-Amidinobenzyl, Pro = Prolin,
Cpa = Diphenylalanin, Phg = Phenylglycin, Asp = Asparaginsäure,
Aze = Azetidin-2-carbonsäure, Pip = Pipecolinsäure

Die Verbindungen der Formel I können als solche oder in Form ihrer Salze mit physiologisch verträglichen Säuren vorliegen. Beispiele für solche Säuren sind: Salzsäure, Zitronensäure, Weinsäure, Milchsäure, Phosphorsäure, Methansulfonsäure, Essigsäure, Ameisensäure, Maleinsäure, Fumarsäure, Maleinsäure, Bernsteinsäure, Hydroxybernsteinsäure, Schwefelsäure, Glutarsäure, Asparaginsäure, Brenztraubensäure, Benzoesäure, Glucuronsäure, Oxalsäure, Ascorbinsäure und Acetylglycin.

Gegenstand der Erfindung sind weiter die Zwischenprodukte der Formel IV 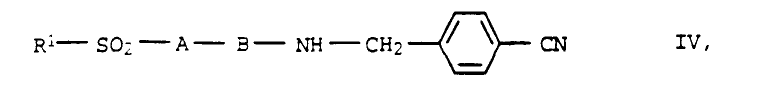 worin R¹, A und B die angegebene Bedeutung besitzen
und die zur Herstellung der Verbindungen I dienen können.

Die Verbindungen I lassen sich ausgehend von der α-Aminosäure H-A-OH bzw. von der N-geschützten cyclischen Aminosäure B-OH nach Reaktionsschema I bzw. II herstellen.

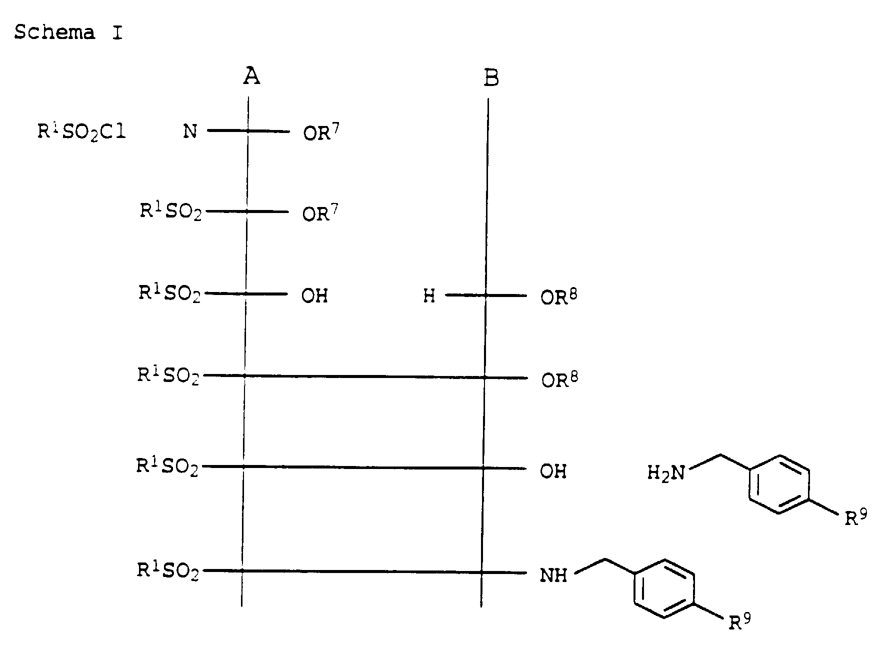

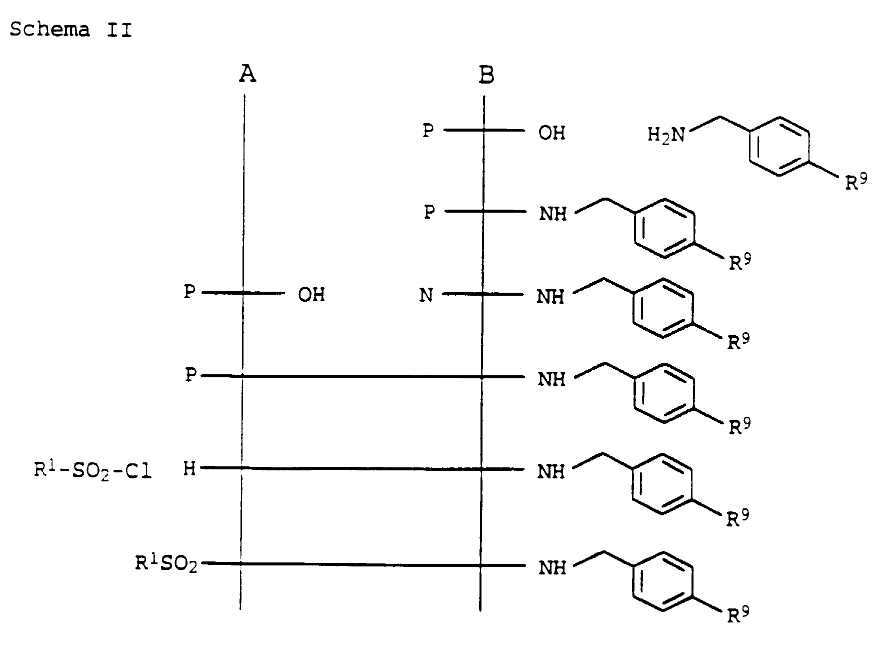

In den vorstehenden Reaktionsschemen bedeuten R⁷ = H oder C₁-C₄-Alkyl, R⁸ = C₁-C₄-Alkyl, bevorzugt Methyl oder t-Butyl, R⁹ = CN oder 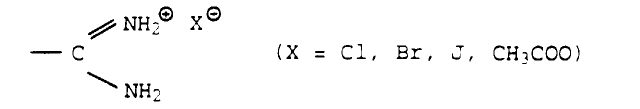 und P eine Schutzgruppe, bevorzugt t-Butoxycarbonyl (Boc) oder Benzyloxycarbonyl (Cbz).

Alternativ können die geschützten Aminosauren P-A-OH und H-B-OR⁸ zum Dipeptid P-A-B-OR⁸ gekuppelt werden und anschließend nach Abspaltung von P mit R¹SO₂Cl bzw. von R⁸ mit p-Cyano- oder p-Amidinobenzylamin umgesetzt werden, wobei die Reaktionsfolge beliebig sein kann.

R¹-SO₂-A-OH kann auch direkt mit para-H-B-NHCH₂C₆H₄R⁹ zum Endprodukt I bzw. Zwischenprodukt IV gekuppelt werden.

Die erforderlichen Kopplungsreaktionen werden nach Standardbedingungen der Peptidchemie durchgeführt (s. M. Bodansky, A. Bodansky "The Practice of Peptide Synthesis", Springer Verlag, 1084).

Boc-Schutzgruppen werden mit HCl/Dioxan oder CF₃COOH/Methylenchlorid, Cbz-Schutzgruppen hydrogenolytisch oder mit HF abgespalten. Die Verseifung von Esterfunktionen erfolgt mit NaOH oder LiOH in einem alkoholischen Lösungsmittel wie Methanol oder Ethanol. t-Butylester werden mit Säuren, z.B. CF₃COOH, verseift.

Die Umsetzung mit den Sulfonylchloriden R¹-SO₂Cl in Gegenwart einer organischen Base wie Triethylamin, Pyridin oder N,N-Diisopropylethylamin erfolgt in organischen Lösungsmitteln wie CH₂Cl₂, THF oder DMF. Im Falle freier Carbonsäurefunktionen wird in Gegenwart wäßriger Alkalimetallhydroxid- oder -carbonat-Lösungen umgesetzt.

Die Herstellung der Amidine aus den Nitrilvorstufen erfolgt nach der klassischen Pinner-Synthese (R. Roger u. D.G. Neilson, Chem. Rev. 1961, 61, 179) oder bevorzugt nach einer modifizierten Pinner-Synthese, die über Imino-thioestersalze als Zwischenstufe abläuft (H. Vieweg u.a., Pharmazie 1984, 39, 226). Die katalytische Hydrierung von N-Hydroxyamidinen, die durch Addition von Hydroxylamin an die Cyanogruppe zugänglich sind, mit Raney Ni bzw. Pd/C in alkoholischen Lösungsmitteln führt ebenfalls zu Amidinen (B.J. Broughton u.a., J. Med. Chem. 1975, 18, 1117).

Die neuen Verbindungen lassen sich zur Therapie und Prophylaxe aller Krankheiten verwenden, bei denen Thrombin eine Rolle spielt. Dies sind besonders thromboembolische Erkrankungen wie Myorkardinfarkt, periphere arterielle Verschlußkrankheit, tiefe Venenthrombose, Lungenembolie und Schlaganfall. Darüber hinaus können sie zur Verhinderung der Reocclusion nach Öffnung arterieller Gefäße durch mechanische Methoden oder Lyse verwendet werden.

Ihr besonderer Vorteil liegt darin, daß sie auch nach oraler Gabe wirksam sind.

Die erfindungsgemäßen Verbindungen können in üblicher Weise oral oder parenteral (subkutan, intravenös, intramuskulär, intraperitoneal, rektal) verabfolgt werden. Die Applikation kann auch mit Dämpfen oder Sprays durch den Nasen-Rachenraum erfolgen.

Die Dosierung hangt vom Alter, Zustand und Gewicht des Patienten sowie von der Applikationsart ab. In der Regel beträgt die tägliche Wirkstoffdosis pro Person zwischen etwa 10 und 2000 mg bei oraler Gabe und zwischen etwa 1 und 200 mg bei parenteraler Gabe. Diese Dosis kann in 2 bis 4 Einzeldosen oder einmalig am Tag als Depotform gegeben werden.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 0,1 bis 99 Gewichtsprozent.

### Beispiel 1

### N-Isopropylsulfonyl-D-phenylalanyl-prolin-(p-amidinobenzyl)amidacetat

a) Boc-D-Phenylalanyl-prolin-(p-cyanobenzyl)amid
   Zu einer Lösung von 5,1 g (14,2 mMol) Boc-D-Phe-Pro-OH und 1,53 g (15,2 mMol) N-Methylmorpholin in 15 ml DMF gab man bei -15°C innerhalb 2 min 2,0 g (14,6 mMol) Chlorameisensäureisobutylester, rührte 10 min nach und gab anschließend eine Lösung von 1,9 g (14,2 mMol) p-Cyanobenzylamin (W. Walter u.a., Ann. 1962, 660, 60) und 1,53 g N-Methylmorpholin in 3 ml DMF zu. Nach 3 h Nachrühren bei -15°C waren laut DC-Kontrolle (CH₂Cl₂/MeOH, 9/1) keine Ausgangsverbindungen mehr nachweisbar.
   Zur Isolierung wurde das Reaktionsgemisch in 200 ml Wasser eingegossen, wobei sich ein Öl abschied, das nach kurzer Zeit erstarrte und nach Zerkleinern abgesaugt wurde. Der noch feuchte Rückstand wurde in einem Gemisch aus 250 ml Essigester und 50 ml Ether gelöst und nacheinander mit einer 5 %igen wäßrigen Zitronensäure-, Bicarbonat- und gesättigten Kochsalzlösung gewaschen. Nach Trocknen über Na₂SO₄ wurde das Lösungsmittel im Vakuum abdestilliert, der Rückstand mit n-Hexan versetzt und anschließend abgesaugt. Eine Umkristallisation aus 50 ml Essigester ergab 5,6 g DC-reines Produkt, Fp. 156 bis 157°C.
b) D-Phenylalanyl-prolin-(p-cyanobenzyl) amid-hydrochlorid
   Die vorstehende Verbindung wurde in 100 ml 5 n HCl in Dioxan gelöst und 3 h bei Raumtemperatur gerührt, wobei das Hydrochlorid ausfiel. Dieses wurde abgesaugt, mit Ether HCl-frei gewaschen und im Vakuum über KOH getrocknet. Man erhielt 4,6 g (95 % d.Th.) weiße Kristalle, Fp. 220-222°C.
c) N-Isopropylsulfonyl-D-phenylalanyl-prolin-(p-cyanobenzyl)amid
   2,05 g (6,05 mMol) des vorstehenden Hydrochlorids wurden in 50 ml CH₂Cl₂ suspendiert. Nach Zugabe von 1,35 g (13,5 mMol) Triethylamin entstand eine Lösung, in die bei 0 bis 5°C 0,9 g (6,1 mMol) Propan-2-sulfonsäurechlorid gelöst in 10 ml CH₂Cl₂ eingetropft wurde. Das Reaktionsgemisch wurde 5 h bei Raum-temperatur nachgerührt und anschließend mit Wasser, 5 %iger Zitronensäure- und 5 %iger NaHCO3-Lösung ausgeschüttelt. Nach Trocknen über Na₂SO₄ und Abdestillieren des Lösungsmittels wurde der zähe, ölige Rückstand aus einem Essigester/Ether-Gemisch (1:1) auskristallisiert.
d) N-Isopropylsulfonyl-D-phenylalanyl-prolin-(p-thioamidinobenzyl)amid
   4,1 g der vorstehenden Verbindung und 4 ml Triethylamin wurden in 40 ml Pyridin gelöst, bei 0°C mit H₂S gesättigt und über Nacht bei Raumtemperatur stehen gelassen. Gemäß DC-Kontrolle (CH₂Cl₂/MeOH, 9/1) war die Umsetzung zum Thioamid vollständig. Zur Isolierung wurde das Pyridin im Vakuum weitgehend abdestilliert, der Rückstand in 250 ml Essigester aufgenommen und mit Kochsalz-, 5 %iger Zitronensäure- und NaHCO₃-Lösung gewaschen. Nach Trocknen und Abdestillieren des Lösungsmittels erhielt man 4,1 g reines kristallines Thioamid.
e) N-Isopropylsulfonyl-D-phenylalanyl-prolin-(p-amidinobenzyl)amid-acetat
   Das Thioamid wurde in 150 ml Aceton gelöst und bei Raumtemperatur nach Zusatz von 7 ml Methyliodid über Nacht stehen gelassen. Nach Abziehen des Lösungsmittels wurde der amorphe Rückstand mit trockenem Ether ausgerührt und anschließend getrocknet. Das S-Methyl-thioimidsäuremethylester-hydroiodid wurde in 50 ml Ethanol gelöst, mit 15 ml 10 %iger Ammoniumacetatlösung versetzt und 3 h auf 60°C erwärmt. Zur Isolierung wurde das Lösungsmittel abgezogen, der Rückstand in 100 ml CH₂Cl₂ gelöst, die unlöslichen Bestandteile abfiltriert und anschließend das CH₂Cl₂ abdestilliert. Durch Digerieren mit einem Essigester-Diethylether-Gemisch wurden die darin löslichen Verunreinigungen abgetrennt. Das verbliebene Iodid-Acetat-Mischsalz wurde in Aceton/Wasser (3/2) gelöst und mittels eines IRA-Acetat-Ionenaustauschers in das reine Acetat überführt, das nach Erwärmen in Acetonitril als kristallines, weißes Pulver, Fp. 148 bis 152°C (Zersetzung), vorlag. FAB-MS (M+H)⁺ = 500.

### Beispiel 2

### N-(Thienyl-2-sulfonyl)-D-phenylalanylprolin-(p-amidinobenzyl)amid-acetat

a) Boc-D-phenylalanyl-prolin-(p-amidinobenzyl)-amid
   Boc-D-Phenylalanyl-prolin-(p-cyanobenzyl)amid(Herstellung s. Beispiel 1a) wurde analog Vorschrift 1c mittels H₂S in das Thioamid und anschließend analog 1d in das Amidin überführt. Das Amidin wurde in Form weißer Kristalle, Fp. 237 bis 239°C, erhalten. FAB-MS (M-H)⁺ = 347.
b) D-Phenylalanyl-prolin-(p-amidinobenzyl)amid-dihydrochlorid
   Analog Beispiel 1b wurde aus vorstehender Verbindung mit 5 n HCl in Dioxan die Boc-Schutzgruppe abgespalten. Das Dihydrochlorid wurde als sehr hygroskopisches Pulver, Fp. 130 bis 140°C, isoliert, FAB-MS (M-H)⁺ = 247.
c) N-(Thienyl-2-sulfonyl)-D-phenylalanyl-prolin-(p-amidinobenzyl)amid-acetat
   Eine Lösung von 3,9 g (12,6 mMol) N-Phenylsulfonyl-D-phenylalanin (Egypt., J. Chem. 1981, 23, 273) in 40 ml THF wurde nach Zugabe von 1,9 g (12,6 mMol) 1-Hydroxybenzotriazol und 3,3 g (25 mMol) Dicyclohexylcarbodiimid 4 h bei Raumtemperatur gerührt. Der ausgefallene Harnstoff wurde abgesaugt und mit wenig THF nachgewaschen.
   Zu diesem Filtrat wurde bei 5°C eine Lösung von 4,1 g (12,6 mMol) N-(p-Amidinobenzyl)prolinamid-dihydrochlorid und 1,6 g Natriumhydrogencarbonat in 6 ml Wasser zugefügt. Nach 48 h Rühren bei Raumtemperatur wurde das Lösungsmittel weitgehend abdestilliert, der Rückstand in Ethanol aufgenommen, von Ungelöstem abfiltriert und erneut eingeengt.
   Der Rückstand wurde über eine Kieselgelsäule mit einem CH₂Cl₂(MeOH/50 %iger Essigsäure-Gemisch (45/5/1,5) gereinigt. Das Eluat der einheitlichen Fraktionen wurde abdestilliert, gegen Ende mit Toluol als Zusatz, und der Rückstand aus 50 ml Aceton unter Zusatz von wenig Wasser umkristallisiert. Man isolierte 3,3 g (48 % d.Th.) Amidinacetat in Form weißer Kristalle, Fp. 95 bis 98°C. FAB-MS (M-H)⁺ = 540.5.

### Beispiel 3

### N-(2-Naphthylsulfonyl)-D-phenylalanylprolin-(p-amidinobenzyl)amid-acetat

a) Boc-Prolin-(p-cyanobenzyl) amid
   276 g BocPro-OSu (0,88 Mol) wurden in 2 l Methylenchlorid bei 0°C vorgelegt. Zu dieser Lösung wurden in Folge 163,9 g 4-Cyanobenzylaminhydrochlorid (0,97 Mol) und 230 ml Diisopropylethylamin (1,34 Mol) gegeben. Die Suspension wurde im auftauenden Eisbad 48 h gerührt und anschließend filtriert. Das Filtrat wurde mit 20 %iger NaHSO₄-Lösung (4 x), gesättigter NaHCO₃-Lösung (3 x) und gesättigter Kochsalzlösung (2 x) extrahiert, getrocknet und einrotiert. Nach Umkristallisation des Rückstands aus Methyl-tert.butylether wurden 261 g (90 %) weiße Kristalle, Fp. 124 bis 125°C, isoliert.
b) N-(4-cyanobenzyl)prolinamid-hydrochlorid
   260 g (0,79 Mol) der vorstehenden Boc-geschützten Verbindung wurden in 1 Liter Diethylether gelöst und nach Zugabe eines Überschusses etherischer HCl-Lösung über Nacht gerührt. Das ausgefallene Hydrochlorid wurde abfiltriert, mit Diethylether HCl-frei gewaschen und anschließend aus Ethanol umkristallisiert. Man erhielt 200 g (95 %) weiße Kristalle, Fp. 209 bis 211°C.
c) N-(2-Naphthylsulfonyl)-D-phenylalanylprolin-(p-cyanobenzyl)amid
   5,9 g (21,3 mMol) des vorstehenden Prolinamid-hydrochlorids wurden in 100 ml DMF gelöst und nacheinander mit 7,8 g (21,3 mMol) N-(2-Naphthylsulfonyl)-D-phenylalanin (A. Bernat u.a., FR 2593812), 2,15 g (21,3 mMol) Triethylamin und 3,25 g (21,3 mMol) 1-Hydroxy-benzotriazol (HOBT) versetzt. Unter Rühren gab man bei 0 bis 5°C eine Lösung von 4,4 g (21,3 mMol) Dicyclohexylcarbodiimid in 30 ml Essigester zu und ließ anschließend 48 h bei Raumtemperatur rühren.
   Nach Absaugen des ausgefallenen Harnstoffs wurde das Lösungsmittel im Vakuum weitgehend abdestilliert, der Rückstand in 200 ml Essigester aufgenommen und nacheinander mit 5 %iger NaHCO₃-Lösung, 4 %iger Zitronensäurelösung und Wasser gewaschen. Nach Trocknen und Abdestillieren des Essigesters wurde der ölige Rückstand in 30 ml CH₂Cl₂ gelöst und durch Zugabe von 50 ml Ether auskristallisiert. Man isolierte 9,1 g (76 %) der gewünschten Verbindung.
d) N- (2-Naphthylsulfonyl)-D-phenylalanylprolin-(p-amidinobenzyl)-amid-acetat
   Unter Feuchtigkeitsausschluß wurden 80 ml Methanol bei 0°C mit Hcl-Gas gesättigt, darin 5,6 g (10 mMol) der vorstehenden Verbindung gelöst und 48 h bei 0°C stehen gelassen. Danach wurde das Lösungsmittel bei 20°C abgezogen, der Rückstand in 20 ml Methanol gelöst und bei 0 bis 5°C zu 80 ml einer NH₃-gesättigten Methanollösung gegeben. Nach 3 h Kochen am Rückfluß wurde die erkaltete Lösung filtriert, das Lösungsmittel abdestilliert und der Rückstand mittels eines Acetat-Ionenaustauschers in das Amidinacetat überführt. Nach Umkristallisation aus Aceton unter Zusatz von wenig Wasser verblieben 5,1 g (80 %) der Titelverbindung als weiße Kristalle, Fp. 221 bis 225°C, FAB-MS (M+H⁺) = 584,5.

### Beispiel 4

### N-(Pyridyl-3-sulfonyl)-D-phenylalanyl-prolin-(p-amidinobenzyl)amid-acetat

a) N-(Pyridyl-3-sulfonyl)-D-phenylalanyl-prolin-(p-cyanobenzyl)amid
   Analog Beispiel 1c wurde D-Phenylalanylprolin-(p-cyanobenzyl)amid-hydrochlorid (Herstellung analog 1a und b) mit Pyridin-3-sulfonsaurechlorid umgesetzt. R_{F} = 0,57 (CH₂Cl₂/MeOH, 9/1).
b) N-(Pyridyl-3-sulfonyl)-D-phenylalanyl-prolin-(p-hydroxyamidinobenzyl) amid-acetat
   2 g der vorstehenden Verbindung, 0,74 g Hydroxylaminhydrochlorid und 2,2 g Triethylamin wurden in 30 ml Ethanol gelöst und unter Stickstoff 2 h bei 60 bis 70°C gerührt. Danach war laut DC-Kontrolle kein Ausgangsmaterial mehr nachweisbar. Das Reaktionsgemisch wurde mit Wasser versetzt und mit Eisessig auf pH 3 bis 4 eingestellt. Die wäßrige Phase wurde mehrmals mit CH₂Cl₂ extrahiert, die vereinigten CH₂Cl₂-Extrakte getrocknet und das Methylenchlorid abdestilliert. Der Rückstand, der noch Essigsäure enthielt, wurde direkt in die Folgereaktion eingesetzt. R_{F} = 0,1 (CH₂Cl₂/MeOH, 9/1).
c) N-(Pyridyl-3-sulfonyl)-D-phenylalanyl-prolin-(p-amidinobenzyl)amid-acetat
   Eine Lösung von 2,4 g (5 mMol) der vorstehenden Verbindung in 40 ml Methanol wurde in Gegenwart von 0,4 g 10 %iger Pd-Kohle 7 h bei 50°C hydriert. Danach wurde der Katalysator abgesaugt, das Lösungsmittel abdestilliert und der Rückstand mit Essigester versetzt und erwärmt. Durch Zugabe von Aceton und wenig Wasser bildete sich eine klare Lösung, aus der beim Abkühlen das Amidacetat auskristallisierte. Man isolierte 1,3 g (49,5 %) weißes Pulver, Fp. 201 bis 202°C, R_{F} 0,28 (CH₂Cl₂/MeOH/50 %ige Essigsäure, 8/2/0,5).

### Beispiel 5

### N- (2-Naphthylsulfonyl)-glycyl-prolin-(p-amidinobenzyl)amid-acetat

a) Boc-Glycyl-prolin-(p-cyanobenzyl)amid
   Zu einer Lösung von 7,0 g (40 mMol) Boc-Glycin in 240 ml Methylenchlorid wurden nacheinander bei 0°C 30 ml Diisopropylethylamin, 10,6 g (40 mMol) N-(p-Cyanobenzyl)prolylamid-hydrochlorid und 32 ml (44 mMol) Propan-phosphonsäureanhydrid (50 %ige Essigesterlösung) zugegeben. Nach 2 h Nachrühren bei 0°C wurde die organische Phase mit 1 n NaOH, Wasser und gesättigter NaCl-Lösung gewaschen, getrocknet und das Lösungsmittel abdestilliert. Man isolierte 14,8 g (96 %) weißes Pulver, R_{F} = 0,57 (CH₂Cl₂/MeOH, 9/1).
   - ¹H-NMR (DMSO-d⁶), δ in ppm:: 1,4 (s, 9H, (CH₃)₃), 1,7-2,2 (m, 4H, CH₂-CH₂), 3,3-3,6 (m, 2H, N-CH₂ vom Prolin), 3,8 (m, 2H, N-CH₂ von Glycin), 4,3-45 (m, 3H, CH und N-CH₂-Ar), 6,8 (m, 1H, BOC-) 7,4-7,5 (m (scheinbares Triplett wegen zweitem Rotamer), 2H, Ar-H), 7,8 (d, 2H, Ar-H) 8,5 und 8,8 (jeweils m, bilden zusammen 1H (zwei Rotamere), NH)
b) H-Glycyl-prolin-(p-cyanobenzyl)amid-hydrochlorid
   Analog 1b wurde aus vorstehender Verbindung die Boc-Gruppe abgespalten. Man isolierte 8 g (64 %) weißes Pulver.
   - ¹H-NMR (DMSO-d⁶), δ in ppm:: 1,7-2,2 (m, 4H, CH₂-CH₂), 3,4-4,0 (m, 4H, N-CH₂ - vom Prolin und Glycin -), 4,2-4,5 (m, 3H, CH und N-CH₂-Ar), 7,5 (d, 2H, Ar), 7,8 (d, 2H, Ar), 8,3 (s, br, 3H, NH₃⁺), 8,9 und 9,2 (jeweils m, bilden zusammen 1H (zwei Rotamere), NH)
c) N-(2-Naphthylsulfonyl)-glycyl-prolin-(p-cyanobenzyl) amid
   Analog 1c wurde aus vorstehender Verbindung durch Umsetzung mit 2-Naphthylsulfonylchlorid 3,3 g weißes Pulver, R_{F} = 0,59 (CH₂Cl₂/MeOH, 9/1), erhalten.
   - ¹H-NMR (DMSO-d⁶), δ in ppm:: 1,6-2,0 (m, 4H, CH₂-CH₂), 3,3-3,5 (m, 2H, N-CH₂ (Prolin)), 3,7 (m, 2H, N-CH₂ (Glycin)), 4,1-4,4 (m, 3H, CH und N-CH₂- Ar), 7,4-8,5 (13H, Aromaten-H und 2NH)
d) N-(2-Naphthylsulfonyl)-glycyl-prolin-(p-thioamidobenzyl)amid
   Vorstehende Verbindung wurde analog 1d in das Thioamid überführt.
   - Ausbeute:: 3,0 g (85 %) gelbliches Pulver
   - ¹H-NMR (DMSO-d⁶), δ in ppm:: 1,5-2,0 (m, 4H, CH₂-CH₂), 3,3-3,5 (m, 2H, N-CH₂ (Prolin)), 3,7 (m, 2H, N-CH₂ (Glycin)), 4,1-4,4 (m, 3H, CH und N-CH₂- Ar), 9,5 (s, 1H, Thioamid), 9,8 (s, 1H, Thioamid)
e) N-(2-Naphthylsulfonyl)-glycyl-prolin-(p-amidinobenzyl)amidacetat
   Die Herstellung erfolgte analog 1e. Es wurden 2,5 g (68 %) Hydroiodid, R_{F} = 0,09 (CH₂Cl₂/MeOH, 9 (1) isoliert und anschließend mittels eines Acetat-Ionenaustauschers (Amberlite) in das Acetat überführt, Reinheit laut HPLC 99 %. FAB-MS (M-H)⁺ = 493.5.
   - ¹H-NMR (DMSO-d⁶), δ in ppm:: 1,6-2,0 (m, 4H, CH₂-CH₂), ~ 3,5 ((m, 2H, N-CH₂ (Prolin) - z.T. vom H₂O-Signal verdeckt), 3,7 (m, 2H, N-CH₂ (Glycin)), 4,1-4,4 (m, 3H, CH und N-CH₂- Ar), 7,3-8,5 (13H, Aromaten-H und NH), ~ 8,4-9,2 (4H, Amidin)

### Beispiel 6

### N-(1-Naphthylsulfonyl)-glycyl-prolin-(p-amidinobenzyl)amid

Analog Beispiel 5 wurde unter Verwendung von 1-Naphthylsulfonylchlorid die Titelverbindung erhalten. FAB-MS (M-H)⁺ = 493.

### Beispiel 7

### N-(n-Hexadecylsulfonyl)-D-phenylalanyl-prolin-(p-amidinobenzyl)amid-acetat

Die Herstellung erfolgte analog Beispiel 1, weißes Pulver, Fp. 194-201°C, FAB-MS (M-H)⁺ = 695.

### Beispiel 8

### N- (n-Butylsulfonyl)-D-phenylalanyl-prolin-(p-amidinobenzyl)amidacetat

Die Herstellung erfolgte analog Beispiel 1, weißes Pulver, Fp. 203-211°C, FAB-MS (M-H)⁺ = 526,5.

### Beispiel 9

### N-(Isopropylaminosulfonyl)-D-phenylalanyl-prolin-(p-amidinobenzyl)-amid-acetat

a) N-(Isopropylaminosulfonyl)-D-phenylalanyl-prolin-(p-cyanobenzyl)-amid
   Analog Beispiel lc wurde D-Phenylalanylprolin-(p-cyanobenzyl)amid-hydrochlorid mit Isopropylaminosulfonsaurechlorid umgesetzt.
b) N-(Isopropylaminosulfonyl)-D-phenylalanyl-prolin-(p-hydroxyamidinobenzyl)-amid
   Die Darstellung dieser Verbindung erfolgte analog Beispiel 4b durch Umsetzung von 9a mit Hydroxylaminhydrochlorid.
c) N-(Isopropylaminosulfonyl)-D-phenylalanyl-prolin-(p-amidinobenzyl)-amid-acetat
   Die Hydrierung von 9b wurde im Methanol/Eisessig/THF-Lösungsmittelgemisch mit Pd/Kohle bei 40°C durchgeführt (13 h). Danach wurde vom Katalysator abgesaugt, die Lösung im Vakuum eingeengt, mehrfach mit Ethanol kodestilliert, der Rückstand in Wasser aufgenommen, die wäßrige Phase 3 mal mit Essigester extrahiert und anschließend die wäßrige Phase, welche das gewünschte Produkt enthielt, lyophilisiert weißer Feststoff, Fp: 199-205°C, FAB-MS: M+H^{⊕}: 515).

### Beispiel 10

### N-(Dimethylaminosulfonyl)-D-phenylalanyl-prolin-(p-amidinobenzyl)-amid-acetat

Die Herstellung erfolgte analog Beispiel 9 (weißer Feststoff, Fp: ab 90°C Zersetzung, FAB-MS: M+H^{⊕}: 501).

### Beispiel 11

### N-Hydroxysulfonyl-D-phenylalanyl-prolin-(p-amidinobenzyl)-amid

Zu einer Lösung von 1,8 g (4,36 mMol) D-Phenylalanyl-prolin-(p-cyanobenzyl)-amid-hydrochlorid und 1,68 g (13,0 mMol) Diisopropylethylamin in 20 ml DCM wurden 0,58 g (0,33 ml, 5 mMol) Chlorsulfonsäure in 10 ml DCM langsam bei 20°C unter Kühlung zugetropft. Nach 30 min Rühren bei Raumtemperatur wurde mit DCM auf 100 ml verdünnt, zunächst mit 2 m HCl, dann zweimal mit 10 ml Wasser extrahiert, die organische Phase über Magnesiumsulfat getrocknet und im Vakuum einrotiert. Man erhielt 2,0 g N-Hydroxysulfonyl-D-phenylalanyl-prolin-(p-cyanobenzyl)-amid als Rohprodukt, welches ohne weitere Reinigung in nachfolgender Reaktion umgesetzt wurde.

2,0 g des genannten Rohprodukts wurden zusammen mit 0,9 g (13 mMol) Hydroxylaminhydrochlorid und 2,5 ml Diisopropylamin in 50 ml Ethanol über Nacht bei Raumtemperatur gerührt, anschließend eingeengt und die flüchtigen Bestandteile im Hochvakuum in 1 h bei 50°C entfernt. Aufgrund der hohen Wasserlöslichkeit des Produkts war eine extraktive Aufarbeitung nicht möglich. Das Rohprodukt (1,8 g) wurde direkt in der nachfolgenden Hydrierung eingesetzt.

Die Hydrierung des Rohprodukts erfolgte unter leichtem Wasserstoffüberdruck in einem Gemisch aus 40 ml Methanol und 5 ml Eisessig mit einer Spatelspitze 10 %igem Palladium auf Kohle bei 50°C. Nach 5,5 h wurde der Katalysator abfiltriert, die Lösung im Vakuum einrotiert und mehrfach mit Methanol und Toluol kodestilliert. Nach mehrmaligem Ausrühren des entstandenen Produkts aus DCM erhielt man 1,5 g (73 % der Theorie über 3 Stufen) sauberes N-Hydroxysulfonyl-D-phenylalanyl-prolin(p-amidinobenzyl)-amid, welches nach NMR als Betain vorlag. Fp: 220-224°C, weißes Pulver, FAB-MS: 474 (M-H)⁺.

Analog Beispiel 4 wurden die nachstehenden Verbindungen hergestellt:

### Beispiel 12

### N-Trifluormethylsulfonyl-D-phenylalanyl-prolin-(p-amidinobenzyl)-amid acetat

Weiße Kristalle, Fp: 240-242°C (Zersetzung), FAB-MS: 526 (M-H)⁺.

### Beispiel 13

### N- (β,β,β-Trifluorethylsulfonyl)-D-phenylalanyl-prolin-(p-amidinobenzyl)-amid-acetat

Weiße Kristalle, Fp: 87-89°C (amorph), FAB-MS: 540 (M-H)⁺.

### Beispiel 14

### N-(n-Octylsulfonyl)-D-phenylalanyl-prolin-(p-amidinobenzyl)-amidacetat

Weiße, amorphe Kristalle, FAB-MS: 570 (M-H)⁺.

### Beispiel 15

### N-Methylsulfonyl-(D,L)-diphenylalanyl-prolin-(p-amidinobenzyl)amid-acetat

Weiße, amorphe Kristalle, FAB-MS: 548 (M-H)⁺.

### Beispiel 16

### N-Methylsulfonyl-di(4-chlorphenyl)alanyl-prolin-(p-amidinobenzyl)-amid-acetat

Weiße, amorphe Kristalle, FAB-MS: 617 (M-H)⁺.

### Beispiel a

Auf einer Tablettenpresse werden in üblicher Weise Tabletten folgender Zusammensetzung gepreßt:
100 mg Substanz des Beispiels 2
240 mg Maisstärke
27 mg Gelatine
90 mg Milchzucker
4,5 mg Aerosil® (chemisch reine Kieselsäure in submikroskopisch feiner Verteilung)
0,5 mg Magnesiumstearat
4,5 mg Talcum

### Beispiel b

In üblicher Weise werden Dragees folgender Zusammensetzung hergestellt:
200 mg Substanz des Beispiels 3
300 mg Kernmasse
350 mg Verzuckerungsmasse

Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60: 40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

### Beispiel c

100 g Substanz des Beispiels 1 werden in 5000 ml Wasser unter Zusatz von NaCl gelöst und mit 0,1 N NaOH auf pH 6,0 eingestellt, so daß eine blutisotonische Lösung entsteht. Jeweils 5 ml dieser Lösung werden in Ampullen gefüllt und sterilisiert.

## Patentansprüche

1. Verbindungen der Formel I 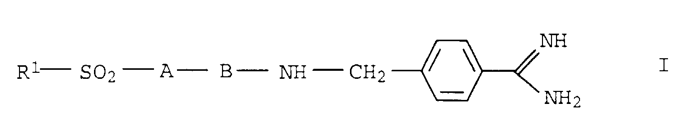 sowie deren Stereoisomeren und deren Salze mit physiologisch verträglichen Säuren, worin die Substituenten folgende Bedeutungen besitzen:
R¹ C₁-C₂₀-Alkyl, C₁-C₃-Fluoralkyl, C₃-C₈-Cycloalkyl, Aryl-C₁-C₁₀-alkyl, Aryl, Heteroaryl, OH oder R³R²N, wobei R² und R³ gleich oder verschieden sind und Wasserstoff, C₁-C₁₀-Alkyl, Aryl, Aryl-C₁-C₁₀-alkyl oder zusammen eine C₂-C₇-Alkylenkette, an die gegebenenfalls ein Aryl- oder Heteroarylrest ankondensiert ist oder die ein Heteroatom (O, S, NH bzw. substituiertes N) enthalten kann, bedeuten,
A ein α-Aminosäurerest der Formel II 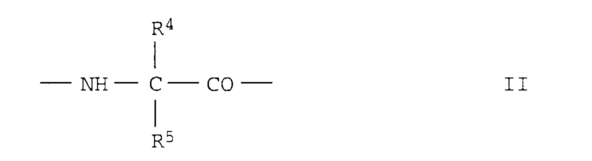 worin
R⁴ Wasserstoff, C₁-C₈-Alkyl, C₃-C₇-Cycloalkyl, Aryl oder Aryl-C₁-C₃-alkyl,
R⁵ Wasserstoff, C₁-C₈-Alkyl, C₃-C₇-Cycloalkyl, C₃-C₇-Cycloalkyl-C₁₋₃-alkyl, Aryl, Aryl-C₁-C₃-alkyl, Di(C₃-C₇-Cycloalkyl)-methyl oder Diphenylmethyl oder - falls R⁴ = H - einen C₁-C₈-Alkylrest, in dem ein Wasserstoffatom durch OR⁶ oder CO-OR⁶ (R⁶ = Wasserstoff, C₁-C₈-Alkyl oder Aryl-C₁-C₃-alkyl) ersetzt ist oder
R⁴ und R⁵ zusammen eine C₂-C₆-Alkylenkette, die einen ankondensierten Arylrest enthalten kann,
bedeuten,
B ein cyclischer α-Aminosäurerest der Formel III 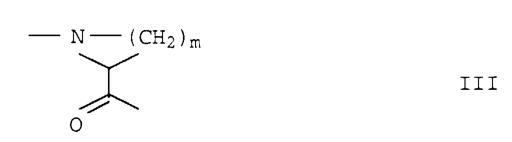 worin m die Zahl 2, 3 oder 4 bedeutet und ein Wasserstoff am Ring durch eine Hydroxy- oder C₁-C₃-Alkylgruppe und - falls m = 3 oder 4 ist - eine CH₂-Gruppe im Ring durch Sauerstoff, Schwefel, NH- oder N-C₁-C₄-Alkyl und/oder zwei benachbarte Wasserstoffatome durch eine Doppelbindung ausgetauscht sein können.

2. Verbindungen der Formel I nach Anspruch 1, wobei R¹ die folgende Bedeutung besitzt:
R¹ OH oder R³R²N, wobei R² und R³ gleich oder verschieden sind und Wasserstoff, C₁-C₁₀-Alkyl, Aryl, Aryl-C₁-C₁₀-alkyl oder zusammen eine C₂-C₇-Alkylenkette, an die gegebenenfalls ein Aryl- oder Heteroarylrest ankondensiert ist oder die ein Heteroatom (O, S, NH bzw. substituiertes N) enthalten kann, bedeuten,

3. Verbindungen der Formel I nach Anspruch 1, wobei R¹, R⁴ und R⁵ und B folgende Bedeutungen besitzen:
R¹ C₁-C₂₀-Alkyl, C₁-C₃-Fluoralkyl, C₃-C₈-Cycloalkyl, Aryl-C₁-C₁₀-alkyl, Aryl oder Heteroaryl bedeuten,
R⁴ C₁-C₈-Alkyl, C₃-C₇-Cycloalkyl, Aryl oder Aryl-C₁-C₃-alkyl,
R⁵ C₁-C₈-Alkyl, C₃-C₇-Cycloalkyl, C₃-C₇-Cycloalkyl-C₁₋₃-alkyl, Aryl, Aryl-C₁-C₃-alkyl, Di(C₃-C₇-Cycloalkyl)-methyl oder Diphenylmethyl oder
R⁴ und R⁵ zusammen eine C₂-C₆-Alkylenkette, die einen ankondensierten Arylrest enthalten kann,
bedeuten,
B einer der Reste

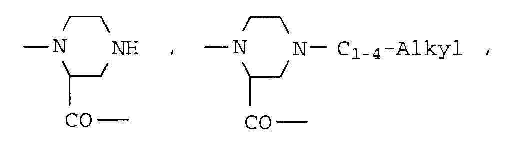

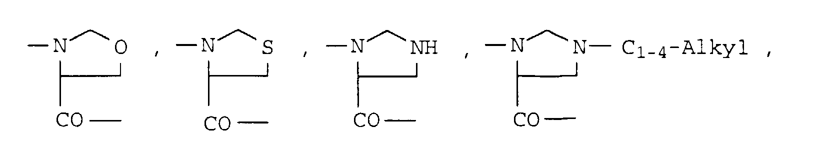

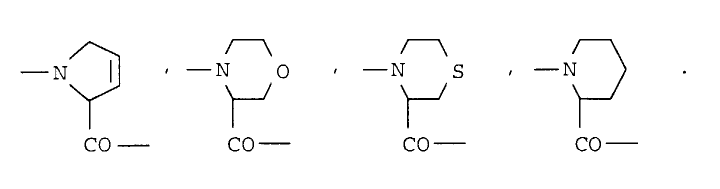

4. Verbindungen der Formel I nach Anspruch 1, wobei R¹ A und B folgende Bedeutungen besitzen:
R¹ C₁-C₂₀-Alkyl, C₁-C₃-Fluoralkyl, C₃-C₈-Cycloalkyl, Aryl-C₁-C₁₀-alkyl, Aryl, Heteroaryl oder OH bedeuten,
A ein α-Aminosäurerest der Formel 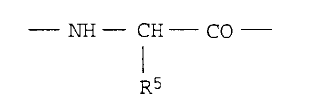 worin
R⁵ einen C₁-C₈-Alkylrest, in dem ein Wasserstoffatom durch OR⁶ oder CO-OR⁶ (R⁶ = Wasserstoff, C₁-C₈-Alkyl oder Aryl-C₁-C₃-alkyl) ersetzt ist,
bedeutet,
B einer der Reste

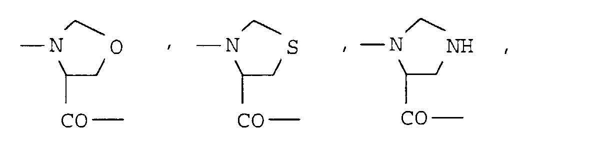

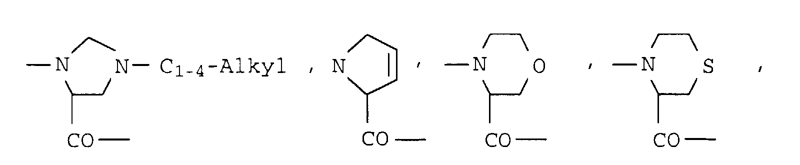

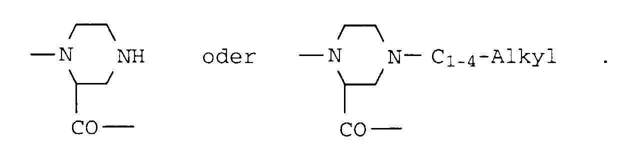

5. Verbindungen der Formel I nach Anspruch 1, wobei R¹, A und B folgende Bedeutungen besitzen:
R¹ C₁-C₂₀-Alkyl, C₁-C₃-Fluoralkyl, C₃-C₈-Cycloalkyl, Aryl-C₁-C₁₀-alkyl, Aryl, Heteroaryl oder OH bedeuten,
A ein α-Aminosäurerest der Formel 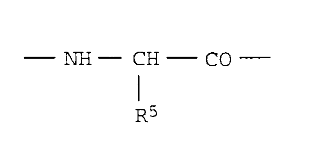 worin
R⁵ einen C₁-C₈-Alkylrest, in dem ein Wasserstoffatom durch OR⁶ oder CO-OR⁶ (R⁶ = Wasserstoff oder C₁-C₈-Alkyl) ersetzt ist, bedeutet,
B ein cyclischer α-Aminosäurerest der Formel III 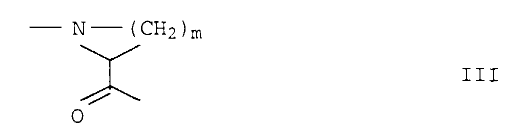 worin m die Zahl 2, 3 oder 4 bedeutet und ein Wasserstoff am Ring durch eine Hydroxy- oder C₁-C₃-Alkylgruppe ausgetauscht ist.

6. Verbindungen der Formel I gemäß einem der Ansprüche 1 bis 5 zur Verwendung bei der Therapie und Prophylaxe von Krankheiten.

7. Verwendung von Verbindungen der Formel I gemäß einem der Ansprüche 1 bis 5 zur Herstellung von Arzneimitteln zur Behandlung von thromboembolischen Erkrankungen oder zur Verhinderung von Reocclusion nach Öffnung arterieller Gefäße durch mechanische Methoden oder Lyse.

## Claims

1. A compound of the formula I 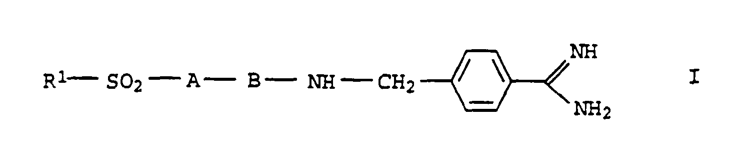 and its stereoisomers and its salts with physiologically tclerated acids, in which the substituents have the following meanings:
R¹ C₁-C₂₀-alkyl, C₁-C₃-fluoroalkyl, C₃-C₈-cycloalkyl, aryl-C₁-C₁₀-alkyl, aryl, hetaryl, OH or R³R²N, where R² and R³ are identical or different and are hydrogen, C₁-C₁₀-alkyl, aryl, aryl-C₁-C₁₀-alkyl or together are a C₂-C₇-alkylene chain to which an aryl or hetaryl radical can be fused or which can contain a hetero atom (O, S, NH or substituted N),
A an α-amino acid residue of the formula II 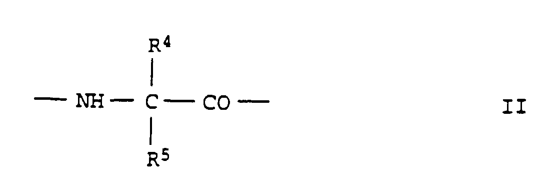 in which
R⁴ is hydrogen, C₁-C₈-alkyl, C₃-C₇-cycloalkyl, aryl or aryl-C₁-C₃-alkyl,
R⁵ is hydrogen, C₁-C₈-alkyl, C₃-C₇-cycloalkyl, C₃-C₇-cycloalkyl-C₁₋₃-alkyl, aryl, aryl-C₁-C₃-alkyl, di(C₃-C₇-cycloalkyl)methyl or diphenylmethyl or - if R⁴ = H - a C₁-C₈-alkyl radical in which one hydrogen atom is replaced by OR⁶ or CO-OR⁶ (R⁶ = hydrogen, C₁-C₈-alkyl or aryl-C₁-C₃-alkyl), or
R⁴ and R⁵ together are a C₂-C₆-alkylene chain, which may contain a fused-on aryl radical,
B a cyclic α-amino acid residue of the formula III 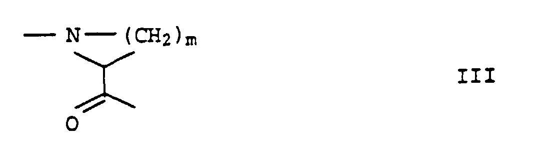 in which m is 2, 3 or 4, and one hydrogen on the ring can be replaced by a hydroxyl or C₁-C₃-alkyl group and - if m = 3 or 4 - a CH₂ group in the ring can be replaced by oxygen, sulfur, NH-or N-C₁-C₄-alkyl and/or two adjacent hydrogen atoms can be replaced by a double bond.

2. Compound of the formula I as claimed in claim 1, where R¹ has the following meaning:
R¹ OH or R³R²N, where R² and R³ are identical or different and are hydrogen, C₁-C₁₀-alkyl, aryl, aryl-C₁-C₁₀-alkyl or together are a C₂-C₇-alkylene chain to which an aryl or hetaryl radical can be fused or which can contain a hetero atom (O, S, NH or substituted N).

3. A compound of the formula I as claimed in claim 1, where R¹, R⁴ and R⁵ and B have the following meanings:
R¹ C₁-C₂₀-alkyl, C₁-C₃-fluoroalkyl, C₃-C₈-cycloalkyl, aryl-C₁-C₁₀-alkyl, aryl or hetaryl,
R⁴ C₁-C₈-alkyl, C₃-C₇-cycloalkyl, aryl or aryl-C₁-C₃-alkyl,
R⁵ C₁-C₈-alkyl, C₃-C₇-cycloalkyl, C₃-C₇-cycloalkyl-C₁₋₃-alkyl, aryl, aryl-C₁-C₃-alkyl, di(C₃-C₇-cycloalkyl)methyl or diphenylmethyl or
R⁴ and R⁵ together are a C₂-C₆-alkylene chain, which may contain a fused-on aryl radical,
B one of the radicals

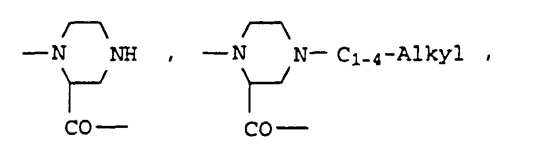

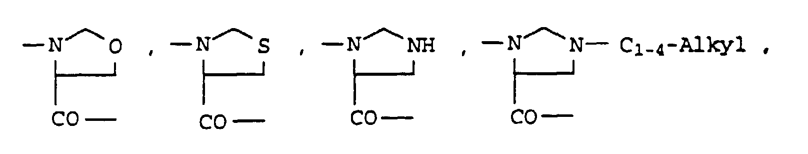

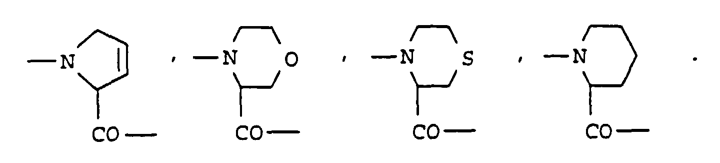

4. A compound of the formula I as claimed in claim 1, where R¹, A and B have the following meanings:
R¹ C₁-C₂₀-alkyl C₁-C₃-fluoroalkyl, C₃-C₈-cycloalkyl, aryl-C₁-C₁₀-alkyl, aryl, hetaryl or OH,
A an α-amino acid residue of the formula 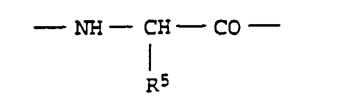 in which
R⁵ is a C₁-C₈-alkyl radical in which one hydrogen atom is replaced by OR⁶ or CO-OR⁶ (R⁶ = hydrogen, C₁-C₈-alkyl or aryl-C₁-C₃-alkyl),
B one of the radicals

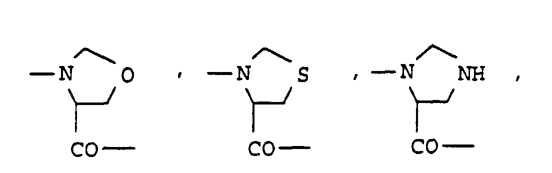

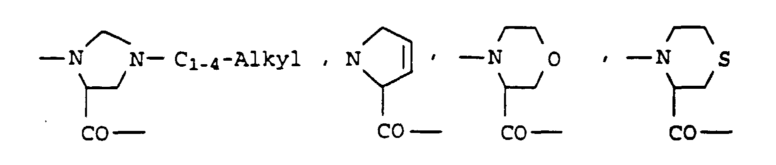

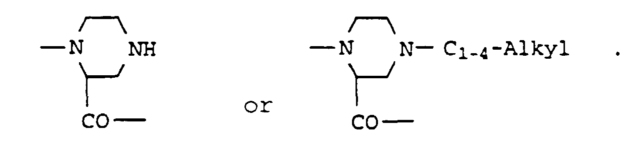

5. A compound of the formula I as claimed in claim 1, where R¹, A and B have the following meanings:
R¹ C₁-C₂₀-alkyl C₁-C₃-fluoroalkyl, C₃-C₈-cycloalkyl, aryl-C₁-C₁₀-alkyl, aryl, hetaryl or OH,
A an α-amino acid residue of the formula 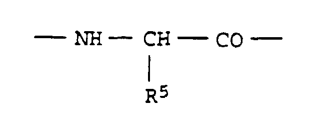 in which
R⁵ is a C₁-C₈-alkyl radical in which one hydrogen atom is replaced by OR⁶ or CO-OR⁶ (R⁶ = hydrogen or C₁-C₈-alkyl),
B a cyclic α-amino acid residue of the formula III 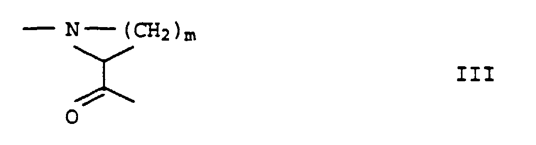 in which m is 2, 3 or 4, and one hydrogen atom on the ring is replaced by a hydroxyl or C₁-C₃-alkyl group.

6. A compound of the formula I as claimed in any of claims 1 to 5 for use in the therapy and prophylaxis of diseases.

7. The use of compounds of the formula I as claimed in any of claims 1 to 5 for producing drugs for treating thromboembolic disorders or for preventing reocclusion after opening of arterial vessels by mechanical methods or lysis.

## Revendications

1. Composés de formule I 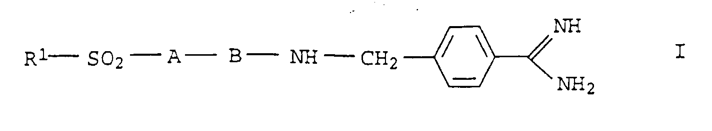 ainsi que leurs stéréoisomères et leurs sels avec des acides physiologiquement acceptables, dans lesquels les substituants ont les significations suivantes:
R¹ alkyle en C₁-C₂₀, fluoroalkyle en C₁-C₃, cycloalkyle en C₃-C₈, arylalkyle(C₁-C₁₀), aryle, hétéroaryle, OH ou R³R²N, tandis que R² et R³ sont identiques ou différents et représentent un radical hydrogène, alkyle en C₁-C₁₀, aryle, arylalkyle(C₁-C₁₀) ou, ensemble, une chaîne alkylène en C₂-C₇, sur laquelle est éventuellement condensé un reste aryle ou hétéroaryle ou qui peut contenir un hétéroatome (O, S, NH ou N substitué),
A un reste d'acide α-aminé de formule II 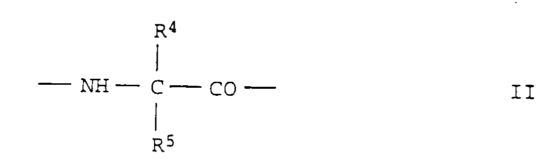 où
R⁴ représente un radical hydrogène, alkyle en C₁-C₈, cycloalkyle en C₃-C₇, aryle ou arylalkyle (C₁-C₃),
R⁵ représente un radical hydrogène, alkyle en C₁-C₈, cycloalkyle en C₃-C₇, cycloalkyle(C₃-C₇)-alkyle(C₁-C₃), aryle, arylalkyle(C₁-C₃), di(cycloalkyl en C₁-C₇)-méthyle ou diphénylméthyle ou - dans le cas où R⁴ = H - un reste alkyle en C₁-C₈, dans lequel un atome d'hydrogène est remplacé par OR⁶ ou CO-OR⁶ (R⁶ = hydrogène, alkyle en C₁-C₈ ou arylalkyle (C₁-C₃)) ou
R⁴ et R⁵ représentent ensemble une chaîne alkylène en C₂-C₆, qui peut contenir un reste aryle condensé,
B un reste d'acide α-aminé cyclique de formule III 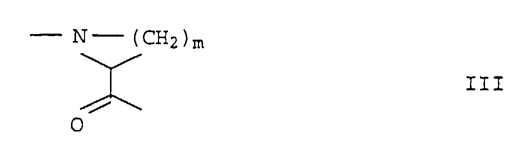 où m représente le nombre 2, 3 ou 4 et un hydrogène sur le cycle peut être remplacé par un groupe hydroxy ou alkyle en C₁-C₃, et - dans le cas où m = 3 ou 4 - un groupe CH₂ dans le cycle peut être remplacé par de l'oxygène, du soufre, NH- ou N-alkyle en C₁-C₄ et/ou deux atomes d'hydrogène voisins peuvent être remplacés par une double liaison.

2. Composés de formule I selon la revendication 1, dans lesquels R¹ possède la signification suivante:
R¹ OH ou R³R²N, tandis que R² et R³ sont identiques ou différents et représentent l'hydrogène ou un groupe alkyle en C₁-C₁₀, aryle, arylalkyle (C₁-C₁₀) ou, ensemble, une chaîne alkylène en C₂-C₇, sur laquelle est éventuellement condensé un reste aryle ou hétéroaryle ou qui peut contenir un hétéroatome (O, S, NH ou N substitué).

3. Composés de formule I selon la revendication 1, dans lesquels R¹, R⁴ et R⁵ et B possèdent les significations suivantes:
R¹ alkyle en C₁-C₂₀, fluoroalkyle en C₁-C₃, cycloalkyle en C₃-C₈, arylalkyle(C₁-C₁₀), aryle ou hétéroaryle,
R⁴ alkyle en C₁-C₈, cycloalkyle en C₃-C₇, aryle ou arylalkyle(C₁-C₃),
R⁵ alkyle en C₁-C₈, cycloalkyle en C₃-C₇, cycloalkyl(C₃-C₇)alkyle(C₁-C₃), aryle, arylalkyle(C₁-C₃), di(cycloalkyl en C₃-C₇)-méthyle ou diphénylméthyle ou
R⁴ et R⁵, ensemble, une chaîne alkylène en C₂-C₆, qui peut contenir un reste aryle condensé,
B l'un des restes

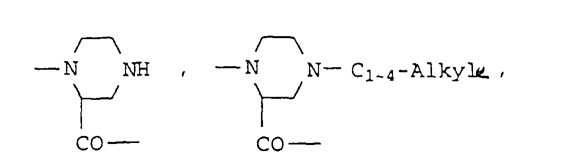

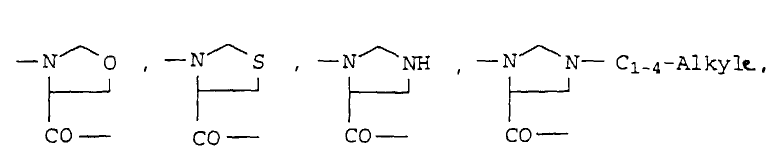

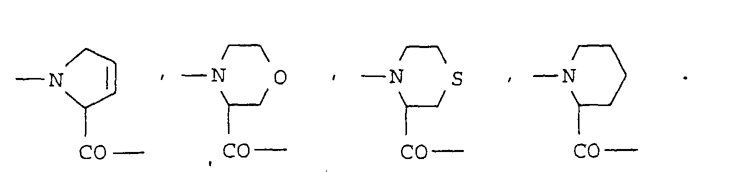

4. Composés de formule I selon la revendication 1, dans lesquels R¹, A et B possèdent les significations suivantes:
R¹ alkyle en C₁-C₂₀, fluoroalkyle en C₁-C₃, cycloalkyle en C₃-C₈, arylalkyle (C₁-C₁₀), aryle ou hétéroaryle ou OH,
A un reste d'acide α-aminé de formule 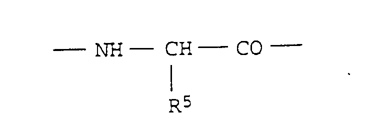 où
R⁵ un reste alkyle en C₁-C₈ dans lequel un atome d'hydrogène est remplacé par OR⁶ ou CO-OR⁶ (R⁶ = hydrogène, alkyle en C₁-C₈ ou arylalkyle (C₁-C₃)),
B l'un des restes

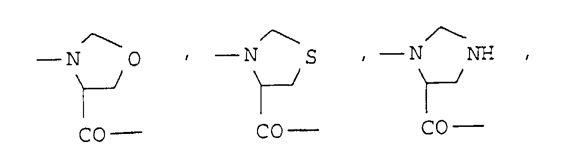

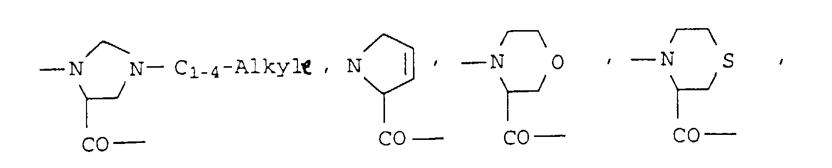

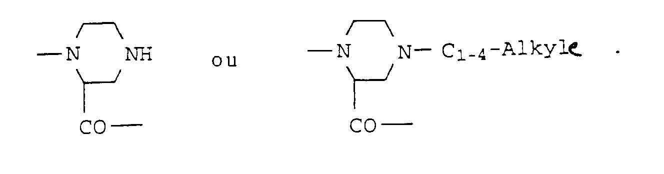

5. Composés de formule I selon la revendication 1, dans lesquels R¹, A et B possèdent les significations suivantes:
R¹ alkyle en C₁-C₂₀, fluoroalkyle en C₁-C₃, cycloalkyle en C₃-C₈, arylalkyle(C₁-C₁₀), aryle ou hétéroaryle ou OH,
A un reste d'acide α-aminé de formule 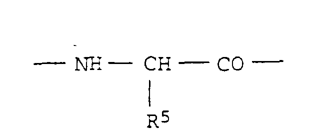 où
R⁵ un reste alkyle en C₁-C₈ dans lequel un atome d'hydrogène est remplacé par OR⁶ ou CO-OR⁶ (R⁶ = hydrogène ou alkyle en C₁-C₈),
B un reste d'acide α-aminé cyclique de formule III 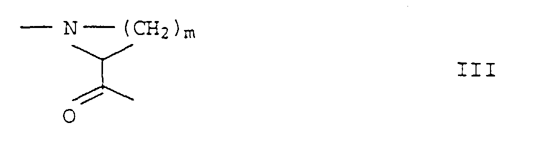 dans lequel m désigne le nombre 2, 3 ou 4 et un hydrogène sur le cycle est remplacé par un groupe hydroxy ou alkyle en C₁-C₃.

6. Composés de formule I selon l'une des revendications 1 à 5 pour l'utilisation dans la thérapie et la prophylaxie de maladies.

7. Utilisation des composés de formule I selon l'une des revendications 1 à 5 pour la préparation de médicaments pour le traitement d'affections thromboemboliques ou pour l'inhibition de la réocclusion après ouverture de conduits artériels par des méthodes mécaniques ou par lyse.
